# DEMANDE DE BREVET EUROPEEN

(11) **EP 0 983 771 A1**
(43) Date de publication de la demande: **08.03.2000**
(21) Numéro de dépôt: 99202713.6
(22) Date de dépôt: 23.08.1999
(51) Int. Cl.: A61M 16/00

(54) **Dispositif d'anesthésie inhalatoire comportant des moyens de surveillance de la pression du gaz dans le circuit annexe**

(30) Priorité: 01.09.1998 FR 9810903
(71) Demandeur: TAEMA, F-92182 Antony Cédex (FR)
(72) Inventeur: Masson, Jean-Philippe, 92370 Chaville (FR)
(74) Mandataire: Le Moenner, Gabriel

(57) **Abrégé**

Appareil d'anesthésie respiratoire comportant :
- un circuit principal (1) d'anesthésie comprenant au moins une canalisation principale (40, 40a, 40b) de gaz à circuit fermé (4) et des moyens (20, 23) de surveillance de la pression, de la composition gazeuse dans ledit circuit principal (1), et
- un circuit annexe (11) d'anesthésie comprenant au moins une canalisation annexe (30) de gaz,
   caractérisé en ce qu'il comporte, en outre, des moyens de mesure (20, 22) reliés audit circuit annexe (11) et permettant de déterminer au moins un paramètre choisi parmi :
- la pression de gaz anesthésique dans au moins une partie du circuit annexe (11),
- la composition du gaz anesthésique acheminé par au moins une partie du circuit annexe (11), et
- la composition du gaz expiré par le patient dans au moins une partie du circuit annexe (11).

## Description

La présente invention se rapporte au domaine de l'anesthésie inhalatoire et, plus particulièrement, à un appareil d'anesthésie inhalatoire comprenant un circuit principal de gaz et un circuit annexe, lequel circuit annexe est équipé de moyens permettant de surveiller, de préférence, en permanence, la pression et le débit du gaz dans ce circuit annexe et la composition des gaz expirés par le patient vers ce circuit annexe.

Les appareils d'anesthésie comportent classiquement un système fermé de canalisations ou analogues, encore appelé circuit principal.

Ce circuit principal est destiné, d'une part, à amener jusqu'aux voies aériennes supérieures du patient des gaz anesthésiques trais issus d'une source de gaz anesthésiques trais et, d'autre part, de récupérer les gaz exhalés par le patient, de permettre une élimination du dioxyde de carbone contenu dans ces gaz exhalés par le patient, de permettre un recyclage et un mélange de ces gaz exhalés purifiés avec les gaz anesthésiques frais avant leur renvoi vers les voies aériennes supérieures du patient.

Ce système fermé fonctionnant en boucle permet de minimiser la quantité de gaz anesthésique frais utilisée pour anesthésier le patient.

De tels dispositifs ont déjà été décrits à maintes reprises dans l'art antérieur; pour plus de détails, on peut se reporter notamment aux documents EP-A-643978, US-A-3687137, EP-A-292615 ou EP-A-266964.

Actuellement, les appareils d'anesthésie inhalatoires existants intègrent sur leur circuit principal des dispositifs permettant de surveiller en permanence la pression des gaz dans ledit circuit principal, c'est-à-dire la pression du gaz anesthésique envoyé vers les voies aériennes supérieures du patient et/ou leur composition, notamment la teneur en oxygène et/ou en constituants halogénés, et/ou la composition des gaz expirés par le patient, notamment la teneur en CO₂ de ces gaz expirés.

Par ailleurs, les appareils d'anesthésie inhalatoire existants comprennent aussi, en général, un circuit annexe relié à la source de gaz anesthésique, lequel circuit annexe est habituellement utilisé pendant la phase d'induction de l'anesthésie, c'est-à-dire au démarrage de l'anesthésie du patient, mais dont l'emploi peut, dans certains cas, se prolonger pendant toute la durée de l'anesthésie proprement-dite, en particulier, en cas de ventilation du patient au moyen d'un masque laryngé.

Or, il est de coutume de ne pas surveiller ce circuit annexe, c'est-à-dire, que la pression du gaz dans le circuit annexe ou sa composition n'est pas suivie en permanence, comme c'est le cas pour le circuit principal d'anesthésie, ainsi que susmentionné.

Dès lors, il se pose un réel problème de sécurité pour le patient lors de l'utilisation du circuit annexe pendant la phase d'induction de l'anesthésie, voire même pendant toute la durée de l'anesthésie, ce qui n'est pas acceptable compte tenu du risque potentiel pour le patient.

Le but de la présente invention est donc de proposer un appareil d'anesthésie inhalatoire permettant de résoudre le problème précité et donc de minimiser les risques pour le patient.

La présente invention concerne alors un appareil d'anesthésie respiratoire comportant :
- un circuit principal d'anesthésie comprenant au moins une canalisation principale de gaz à circuit fermé, et des moyens de surveillance de la pression et/ou de la composition du gaz dans ledit circuit principal,
- un circuit annexe d'anesthésie comprenant au moins une canalisation annexe de gaz,
   caractérisé en ce qu'il comporte, en outre, des moyens de surveillance supplémentaires reliés au moins audit circuit annexe et permettant de déterminer au moins un paramètre choisi parmi :
- la pression de gaz anesthésique dans au moins une partie du circuit annexe,
- la composition du gaz anesthésique acheminé par au moins une partie du circuit annexe, et
- la composition du gaz expiré par le patient dans au moins une partie du circuit annexe.

Selon le cas, l'appareil selon l'invention peut comprendre l'une ou plusieurs des caractéristiques suivantes:
- la canalisation annexe de gaz comporte à l'une de ses extrémités des moyens de raccordement de sortie permettant de relier, au moins temporairement, ladite canalisation annexe aux voies aériennes supérieures d'un patient, lesdits moyens de surveillance supplémentaires étant reliés à la canalisation annexe du circuit annexe, en amont des moyens de raccordement de sortie, de préférence à proximité immédiate desdits moyens de raccordement de sortie;
- les moyens de surveillance supplémentaires comprennent au moins un capteur de pression et/ou au moins un dispositif d'analyse de gaz;
- les moyens de surveillance supplémentaires sont connectés à des moyens de visualisation;
- les moyens de raccordement de sortie sont choisis parmi les masques respiratoires et les sondes d'intubation;
- les moyens de surveillance et les moyens de surveillance supplémentaires sont reliés à de même moyens de visualisation;
- les moyens de surveillance et les moyens de surveillance supplémentaires comprennent un dispositif d'analyse de gaz commun.
   L'invention concerne en outre un procédé de surveillance d'au moins un paramètre choisi parmi la pression de gaz et la composition de gaz inhalé et/ou expiré, à l'intérieur d'au moins une partie d'un circuit annexe d'anesthésie d'un appareil d'anesthésie inhalatoire comportant :
- un circuit principal d'anesthésie comprenant au moins une canalisation principale de gaz à circuit fermé, et
- ledit circuit annexe comprenant au moins une canalisation annexe de gaz,
   dans lequel :
- on alimente au moins une partie du circuit annexe avec un gaz anesthésique,
- on détermine, dans au moins une partie du circuit annexe, au moins un paramètre choisi parmi la pression de gaz anesthésique, la composition du gaz anesthésique avant et/ou après exhalation par le patient, dans au moins une partie du circuit annexe;
- on détermine, en outre, au moins un paramètre choisi parmi la pression de gaz anesthésique et la composition du gaz anesthésique avant et/ou après exhalation par le patient, dans au moins une partie du circuit principal;
- on visualise au moins un paramètre choisi parmi la pression de gaz anesthésique et la composition du gaz anesthésique avant et/ou après exhalation par le patient, dans le circuit principal et/ou le circuit annexe au moyen de moyens de visualisation.
   Selon le cas, le procédé selon l'invention peut comprendre l'une ou plusieurs des caractéristiques suivantes:
- la détermination du paramètre est réalisée en continu, de manière à obtenir une image complète dudit ou desdits paramètres au cours du temps.

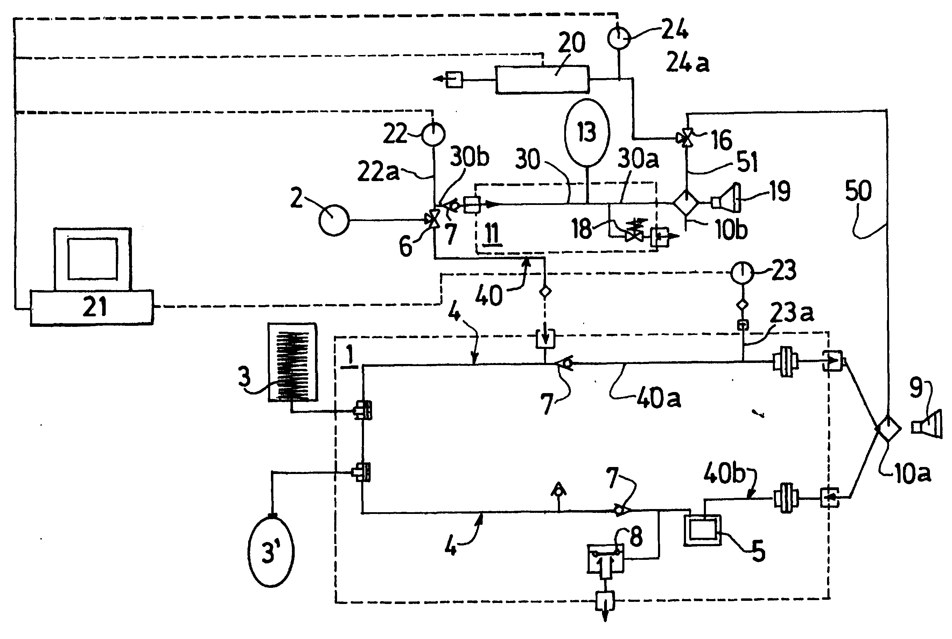

La présente invention va maintenant être décrite plus en détail en référence à la figure unique annexée, laquelle représente un appareil d'anesthésie inhalatoire conforme à la présente invention comprenant, d'une part, un circuit principal 1 d'anesthésie et, d'autre part, un circuit annexe 11 d'anesthésie.

Plus précisément, le circuit principal d'anesthésie 1 comporte une canalisation principale 40 de gaz anesthésique à circuit fermé 4, comprenant une branche d'inspiration 40a et une branche d'expiration 40b, lesdites branches 40a et 40b formant le circuit fermé ou circuit en boucle.

La partie aval de la branche inspiratoire 40a est reliée à un raccord intermédiaire 10a permettant en outre le prélèvement des gaz inhalés pour analyse, ledit raccord intermédiaire 10a étant lui-même relié aux voies aériennes supérieures d'un patient (non représenté) par l'intermédiaire d'un moyen de raccordement de sortie 9 ou embout de raccordement, par exemple un masque respiratoire ou une sonde d'intubation, permettant de distribuer un gaz anesthésique aux voies respiratoires supérieures dudit patient.

En outre, la branche inspiratoire 40a est reliée par son extrémité amont à un dispositif de commutation 6 d'une source de gaz anesthésiques 2, par exemple un mélange gazeux contenant de l'oxygène, de l'azote et des produits halogénés, de manière à pouvoir notamment alimenter un ballon réservoir 3.

Par ailleurs, le circuit fermé 4 comporte également une branche expiratoire 40b dont l'extrémité amont se raccorde à l'extrémité aval de la branche inspiratoire 40a au niveau du raccord intermédiaire 10a, de manière à recueillir les gaz expirés par le patient, et dont l'extrémité aval se raccorde à l'extrémité amont de la branche inspiratoire 40a à proximité du ballon-réservoir 3, de manière à constituer le circuit fermé 4.

En fait, le circuit principal 1 comporte deux ballons-réservoirs 3 et 3', le ballon-réservoir 3 étant dédié à un mode ventilation mécanique et le ballon-réservoir 3' étant dédié à un mode ventilation manuel.

La branche expiratoire 40b comporte un dispositif 5 de purification permettant d'éliminer au moins une partie du dioxyde de carbone (CO₂) contenu dans les gaz expirés par le patient, par exemple une cartouche d'adsorbant.

La branche expiratoire 40b comporte également un échappement 8 vers l'atmosphère destiné à pallier toute surpression dans le circuit principal 1.

En outre, des clapets anti-retour 7 sont aménagés sur lesdites branches inspiratoire 40a et expiratoire 40b.

La pression du gaz dans la canalisation 40 principale est déterminée au moyen d'un capteur de pression 23 relié (en 23a) à ladite canalisation principale 40; la prise de pression étant effectuée en aval de ladite canalisation principale 40, c'est-à-dire, de préférence, à proximité de la pièce de raccordement de sortie 9.

Par ailleurs, un analyseur 20 de gaz permet de contrôler la composition de gaz anesthésique dans ladite canalisation 40, avant inhalation du gaz anesthésique par le patient et/ou après exhalation par le patient des gaz expirés riches en dioxyde de carbone. La détermination de la concentration des gaz dans la canalisation 40 est obtenue grâce à un prélèvement de gaz, de préférence en continu, au niveau du raccord intermédiaire 10a, lequel gaz est acheminé jusqu'à l'analyseur de gaz 20 pour y être analysé.

Les informations recueillies par le capteur de pression 23 et l'analyseur 20 sont transmises vers un écran vidéo de visualisation 21, sur lequel la composition des gaz et/ou la pression peuvent être suivies en permanence par le personnel hospitalier, tel le médecin ou analogue.

L'appareil selon l'invention comporte, en outre, un circuit annexe 11 d'anesthésie comprenant une canalisation annexe 30 de gaz susceptible d'être reliée, par son extrémité amont 30b et via le dispositif de commutation 6, à la source 2 de gaz frais et, par son extrémité aval 30a, à un raccord intermédiaire 10b lui-même susceptible d'être relié au patient par l'intermédiaire de moyens de raccordement 19 de sortie, tel un masque respiratoire ou une sonde d'intubation.

Ce circuit annexe 11 est destiné à être utilisé pendant la phase d'induction de l'anesthésie, c'est-à-dire au début de celle-ci, ou, selon le cas, pendant toute la durée de l'anesthésie.

Comme on peut le voir sur la figure, le circuit annexe 30 comporte un clapet anti-retour 7, en aval duquel se raccorde un deuxième ballon-réservoir 13 de gaz anesthésique et une valve d'échappement 18 vers l'atmosphère.

Des moyens de surveillance supplémentaires 20 et 22 reliés au circuit annexe 11, permettent de déterminer, selon le cas, la pression et/ou la composition du gaz dans la canalisation annexe 30.

La détermination de la concentration du gaz inhalé et/ou expiré par le patient dans la canalisation annexe 30 est réalisée grâce à un prélèvement de gaz, de préférence en continu, au niveau ou à proximité du raccord intermédiaire 10b, lequel gaz est ensuite analysé dans l'analyseur 20, qui peut être le même analyseur que celui utilisé pour déterminer la composition des gaz inhalés et expirés dans le circuit principal 1 de l'appareil ou, selon le cas, un analyseur différent.

De préférence, il s'agit d'un même analyseur 20, relié soit à un dispositif de commutation 16 permettant de sélectionner, de préférence électroniquement, la ligne de prélèvement 50 ou 51 correcte, c'est-à-dire la ligne sur laquelle doit être effectué le prélèvement du gaz, soit à une seule ligne de prélèvement 50 reliée aux raccords intermédiaires 10a ou 10b, et placée par le personnel hospitalier, selon le cas, soit entre le circuit annexe 30 et les moyens de raccord de sortie 19, soit entre la canalisation 40 et les moyens de raccord de sortie 9.

En outre, la pression du gaz anesthésique dans le circuit annexe 11 peut être déterminée au moyen d'un capteur de pression 22 dont la prise de pression est située en amont ou, le cas échéant, en aval du clapet anti-retour 7.

Les informations de pression et/ou de composition du gaz dans le circuit annexe 11 sont ensuite transmises vers les moyens de visualisation 21 où elles sont affichées et peuvent être suivies en permanence par le personnel médical.

Pour obtenir une représentation fiable et précise de la pression du gaz dans le circuit annexe 11, il est souhaitable de retrancher la perte de charge engendrée par le débit de gaz frais dans le circuit annexe 11 de la valeur de pression mesurée dans ce circuit annexe 11.

De plus, un capteur de pression 24, faisant office de moyens de contrôle de sécurité, placé sur la ligne de prélèvement 50 ou 51 de l'analyseur de gaz permet de mesurer, selon le cas, la pression (en 24a) dans la canalisation annexe 30 ou la canalisation principale 40 et apporte ainsi une redondance sur les mesures effectuées par les moyens de surveillance 22 et 23 ce qui renforce la sécurité du dispositif.

Une information fiable peut être obtenue en retranchant la perte de charge engendrée par le débit d'aspiration maintenu par l'analyseur de gaz 20 dans la ligne de prélèvement 50 ou 51, à la valeur de pression mesurée par le capteur 24.

L'alimentation du circuit annexe 11 en gaz frais anesthésique, délivré par la source 2 de gaz, est obtenue par actionnement de moyens de commutation 6, situés à l'extrémité amont 30b de la canalisation annexe 30.

L'appareil d'anesthésie selon la présente invention permet contrairement à un dispositif classique existant actuellement, de suivre en permanence, non seulement la pression et la composition des gaz dans le circuit principal, mais aussi la composition et la pression des gaz dans le circuit annexe équipant ce ventilateur d'anesthésie.

## Revendications

1. Appareil d'anesthésie respiratoire comportant :
- un circuit principal (1) d'anesthésie comprenant au moins une canalisation principale (40, 40a, 40b) de gaz à circuit fermé (4) et des moyens (20, 23) de surveillance de la pression et de la composition des gaz dans ledit circuit principal (1), et
- un circuit annexe (11) d'anesthésie comprenant au moins une canalisation annexe (30) de gaz,
caractérisé en ce qu'il comporte, en outre, des moyens de surveillance supplémentaires (20, 22) reliés au moins audit circuit annexe (11) et permettant de déterminer au moins un paramètre choisi parmi :
- la pression de gaz anesthésique dans au moins une partie du circuit annexe (11),
- la composition du gaz anesthésique acheminé par au moins une partie du circuit annexe (11), et
- la composition du gaz expiré par le patient dans au moins une partie du circuit annexe (11).

2. Appareil selon la revendication 1, caractérisé en ce que la canalisation annexe (30) de gaz comporte à l'une de ses extrémités (30a) des moyens de raccordement de sortie (19) permettant de relier, au moins temporairement, ladite canalisation annexe (30) aux voies aériennes supérieures d'un patient, les moyens de surveillance supplémentaires (20, 22) étant reliés à la canalisation annexe (30) du circuit annexe (11), en amont des moyens de raccordement de sortie (19), de préférence à proximité immédiate desdits moyens de raccordement de sortie (19), par exemple par un raccord intermédiaire (10b).

3. Appareil selon l'une des revendications 1 ou 2, caractérisé en ce que les moyens de surveillance supplémentaires (20, 22) comprennent au moins un capteur de pression (22) et/ou au moins un dispositif d'analyse de gaz (20).

4. Appareil selon l'une des revendications 1 à 3, caractérisé en ce que les moyens de surveillance supplémentaires (20, 22) sont connectés à des moyens de visualisation (21), de préférence un moniteur ou un écran vidéo.

5. Appareil selon l'une des revendications 1 à 4, caractérisé en ce que les moyens de raccordement (19) de sortie sont choisis parmi les masques respiratoires ou les sondes d'intubation.

6. Appareil selon l'une des revendications 1 à 5, caractérisé en ce que les moyens de surveillance (20, 23) et les moyens de surveillance supplémentaire (20, 22) sont reliés à de même moyens de visualisation (21).

7. Appareil selon l'une des revendications 1 à 6, caractérisé en ce que les moyens de surveillance (20, 23) et les moyens de surveillance supplémentaires (20, 22) comprennent un dispositif d'analyse de gaz (20) commun.

8. Appareil selon la revendication 6 ou 7, caractérisé en ce que des moyens de contrôle de sécurité (24) sont agencés sur la ligne de prélèvement (50, 51) alimentant l'analyseur de gaz, de manière à permettre de mesurer au moins la pression de gaz dans ladite ligne de prélèvement (50, 51) ou dans ladite canalisation principale (40).

9. Procédé de surveillance d'au moins un paramètre choisi parmi la pression de gaz et la composition de gaz, à l'intérieur d'au moins une partie d'un circuit annexe (11) d'anesthésie d'un appareil d'anesthésie respiratoire comportant :
- un circuit principal (1) d'anesthésie comprenant au moins une canalisation principale (40, 40a, 40b) de gaz à circuit fermé (4) et des moyens (20, 23) de surveillance de la pression, de la composition des gaz dans ledit circuit principal (1), et
- ledit circuit annexe (11) comprenant au moins une canalisation annexe (30) de gaz,
dans lequel :
- on alimente au moins une partie du circuit annexe (11) avec un gaz anesthésique, et
- on détermine à l'aide des moyens de surveillance (20, 22), dans au moins une partie d'au moins ledit circuit annexe (11), au moins un paramètre choisi parmi la pression de gaz anesthésique, la composition du gaz anesthésique avant et/ou après exhalation par le patient, dans au moins une partie du circuit annexe (11).

10. Procédé selon la revendication 9, caractérisé en ce qu'on détermine, en outre, au moins un paramètre choisi parmi la pression de gaz anesthésique et la composition du gaz anesthésique avant et/ou après exhalation par le patient dans au moins une partie du circuit principal (1).

11. Procédé selon la revendication 9 ou 10, caractérisé en ce qu'on visualise au moins un paramètre choisi parmi la pression de gaz anesthésique et la composition du gaz anesthésique avant et/ou après exhalation par le patient, mesuré dans le circuit principal (1) et/ou le circuit annexe (11), au moyen de moyens de visualisation (21).

12. Procédé selon la revendication 11, caractérisé en ce que la détermination du paramètre est réalisée en continu.
